# EUROPEAN PATENT SPECIFICATION

(11) **EP 3 016 578 B1**
(45) Date of publication and mention of the grant of the patent: **01.01.2020**
(21) Application number: 14820085.0
(22) Date of filing: 01.07.2014
(51) Int. Cl.: A61B 5/00, G16H 40/63, G16H 10/60, G16H 50/30, A61B 5/01, A61B 5/024, A61B 5/0408, A61B 5/0478, A61B 5/0492, A61B 5/053, A61B 5/08, A61B 5/11, A61B 5/117, A61B 5/145

(54) **ALGORITHMS FOR PERSONALIZATION OF MONITORING SIGNALS IN REMOTE PATIENT MONITORING SYSTEMS**
ALGORITHMEN ZUR PERSONALISIERUNG VON ÜBERWACHUNGSSIGNALEN BEI ENTFERNTEN PATIENTENÜBERWACHUNGSSYSTEMEN
ALGORITHMES POUR LA PERSONNALISATION DE SIGNAUX DE SURVEILLANCE DANS DES SYSTÈMES DE SURVEILLANCE À DISTANCE DE PATIENTS

(30) Priority: 01.07.2013 US 201361841861 P
(43) Date of publication of application: 11.05.2016
(60) Divisional application: 19191002.5
(73) Proprietor: Mayo Foundation for Medical Education and Research, Rochester, MN 55905 (US)
(72) Inventor: BENNET, Kevin E., Rochester, Minnesota 55902 (US); BRUCE, Charles J., Rochester, Minnesota 55902 (US); FRIEDMAN, Paul A., Rochester, Minnesota 55902 (US); SOMERS, Virend K., Rochester, Minnesota 55902 (US)
(74) Representative: Väänänen, Mikko Kalervo
(86) International application number: PCT/US2014/045026
(87) International publication number: WO 2015/002933

(56) References cited:
- US-A1- 2006 111 642
- US-A1- 2006 247 501
- US-A1- 2007 185 393
- US-A1- 2008 288 026
- US-A1- 2009 234 672
- US-A1- 2010 022 903
- US-A1- 2010 052 915
- US-A1- 2010 286 532
- US-A1- 2011 105 927
- US-A1- 2011 270 117
- US-A1- 2011 270 117
- US-A1- 2012 088 999
- US-A1- 2012 101 349
- US-A1- 2013 116 533

## Description

### BACKGROUND

### 1. Technical Field

This document relates to devices and methods for monitoring patient health parameters using a wearable monitoring device. For example, this document relates to the use of device, methods, and algorithms for the personalization of a remote patient monitoring system.

### 2. Background Information

For a variety of reasons, the importance of remote health monitoring systems, such as in-home monitoring systems, is increasing. Remote health parameter monitoring of ambulatory patients enables doctors to detect or diagnose health problems, such as heart arrhythmias, that may produce only transient symptoms and therefore may not be evident when the patients visit the doctors' offices. Remote health parameter monitoring is a significant tool available to healthcare providers for reducing hospital readmission rates and to track disease progression. The use of monitoring systems can permit a smooth transition from hospital to home care. Steadily increasing healthcare costs and outpatient populations have created a need to maximize time intervals between office visits.

The relentless pressure to reduce costs in the healthcare industry has required the more efficient use of a healthcare professional's services. As a result, many physicians now regularly prescribe home monitoring of such health parameters as blood pressure, heart rate, blood glucose level, and EKG (electrocardiogram) signals. In addition, health insurance providers are increasingly viewing remote health parameter monitoring as a means to reduce in-patient expenses and overall healthcare costs.

US20100286532 of the prior art relates to an apparatus for determining physiological status of an individual. The apparatus determines the physiological status based on heart-related signals of the individual. The apparatus comprises a wearable sensor device to be worn by a user. The wearable sensor device is attached with electrodes, and a processor is connected with the electrodes. The electrodes comprise microneedles for providing better positioning and contact with skin of the individual. The heart-related signals, collected by the electrodes, are processed to determine the physiological status of the individual.

### SUMMARY

This document provides devices and methods for monitoring patient health parameters using a wearable monitoring device. For example, this document provides device, methods, and algorithms for the personalization of a remote patient monitoring system.

In a general aspect, this document features a health parameter monitoring system comprising: a sensor patch in contact with a human skin surface, wherein the sensor patch comprises a plurality of sensors for measuring physiologic or pathologic parameters; a control unit, wherein the control unit is releasably receivable in a cradle of the sensor patch, and wherein the control unit is in electrical communication with the plurality of sensors when the control unit is in the cradle; a cap, wherein the cap is configured to releasably couple with the sensor patch to detain the control unit in the cradle, and wherein the cap includes a user interface that is configured to provide indications related to the functioning of the health parameter monitoring system; and a biometric interface, wherein the biometric interface is configured to detect a biometric characteristic of a user of the health parameter monitoring system.

In various implementations, in response to detecting a particular biometric characteristic of a user the health parameter monitoring system may operate, and in response to not detecting the particular biometric characteristic of a user the health parameter monitoring system may not operate. The particular biometric characteristic may be a fingerprint. The particular biometric characteristic may be a QRS morphology. The particular biometric characteristic may be a bioimpedance.

In another general aspect, this document features a method of operating a health parameter monitoring system. The method comprises: providing the health parameter monitoring system; detecting a particular biometric characteristic of a user; determining whether the detected particular biometric characteristic is sufficiently similar to a template of the particular biometric characteristic; and allowing the health parameter monitoring system to operate in response to determining that the detected particular biometric characteristic is sufficiently similar to the template of the particular biometric characteristic, or not allowing the health parameter monitoring system to operate in response to determining that the detected particular biometric characteristic is not sufficiently similar to the template of the particular biometric characteristic. The health parameter monitoring system comprises: a sensor patch in contact with a human skin surface, wherein the sensor patch comprises a plurality of sensors for measuring physiologic or pathologic parameters; a control unit, wherein the control unit is releasably receivable in a cradle of the sensor patch, and wherein the control unit is in electrical communication with the plurality of sensors when the control unit is in the cradle; a cap, wherein the cap is configured to releasably couple with the sensor patch to detain the control unit in the cradle, and wherein the cap includes a user interface that is configured to provide indications related to the functioning of the health parameter monitoring system; and a biometric interface, wherein the biometric interface is configured to detect a biometric characteristic of a user of the health parameter monitoring system.

Particular embodiments of the subject matter described in this document can be implemented to realize one or more of the following advantages. In some embodiments, an integrated health monitoring system based on acquisition of physiologic and pathologic parameters from sensors can facilitate ambulatory care to promote patient independence and permit a smooth transition from hospital to home care. In some embodiments, the algorithms provided herein can enable enhanced detection of health issues by using personalized templates and algorithms that can identify deviations from normal in the context of an individual patient and in a particular patient context. In some embodiments, the algorithms provided herein can be used to increase the accuracy of the data collected by a health monitoring system by detecting and rejecting some signals as artifact noise. In result, the accuracy and effectiveness of health parameter monitoring systems can be improved, overall healthcare costs can be reduced, and patient health and longevity can be enhanced using the devices and methods provided herein. Unless otherwise defined, all technical and scientific terms used herein have the same meaning as commonly understood by one of ordinary skill in the art to which this invention pertains.

The details of one or more embodiments of the invention are set forth in the accompanying drawings and the description herein. The invention is defined by appended claims 1-6.

### DESCRIPTION OF THE DRAWINGS

Figures 1A-1C are illustrations of a modular external patient monitoring device in accordance with some embodiments provided herein.
Figure 2 is an illustration of a patient wearing the modular external patient monitoring device of Figures IA-IC.
Figure 3 is a side cross-sectional view another modular external patient monitoring device in accordance with some embodiments provided herein.
Figures 4A and 4B are illustrations of additional modular external patient monitoring devices in side cross-sectional views in accordance with some embodiments provided herein.
Figure 5 is a flowchart of a process of using a health monitoring system that includes personalized algorithms to detect abnormalities and artifact in accordance with some examples.

Like reference numbers represent corresponding parts throughout.

### DETAILED DESCRIPTION

This document provides devices and methods for the remote monitoring of patient health parameters. For example, this document provides devices, methods, and algorithms for the personalization of a remote patient monitoring system. In some examples,
the remote health parameter monitoring system includes a wearable component and a separate computing device that can communicate with each other as well as with a remote monitoring service. In some examples, a controller unit in the wearable component performs the personalized algorithms for artifact rejection and for detection of health monitoring events. In other embodiments, the separate computing device performs the personalized algorithms for artifact rejection and for detection of health monitoring events. In some examples, both systems can perform such algorithms. While the methods and algorithms provided herein may be described in the context of particular health parameter monitoring systems, it should be understood that the methods, algorithms, and techniques for personalization of health monitoring systems as described in this document can be applied to other monitoring systems or to the data of other monitoring systems.

In one example, biometric identifiers and associated algorithms are included in remote patient monitoring systems. Such biometric identifiers can personalize a monitor to a particular patient as prescribed by a clinician. Biometric identifiers such as finger print recognition, facial recognition, voice recognition, and other techniques can be used to personalize a health parameter monitor to a particular patient. Such techniques can be used to prevent unintended individuals from using a monitor that is prescribed for use by a particular patient.

In another example, during set-up and initialization, a monitoring device can acquire biometric data from the individual using the monitor, and the data can be compared to stored data that was acquired from the intended patient. Algorithms can be applied to determine whether the individual using the monitor is likely the intended patient or not. Biometric data including but not limited to ECG, QRS morphology, QRS amplitude, QRS duration, PR interval, impedance, and the like can be used for such comparisons.

In yet another example, personal templates for a patient can be created by a clinician or automatically by the monitoring system. The personal templates can be physiological biometric data or a combination of physiological biometric data such as ECG, QRS morphology, QRS amplitude, QRS duration, PR interval, impedance, and the like. The personal templates can be stored in the memory of the monitoring system. Real-time-measured health parameters can be compared with the personal templates in attempt to determine if differences between the real-time parameters and the personal templates exist. If differences exist, the real-time parameter data can be analyzed to determine if the differences are attributable to artifact signal noise. If artifact is not determined to be the cause of the differences, the monitoring system can initiate corrective actions related to the real-time health parameter data such as triggering an alarm, collecting additional data, recording an event, notifying a monitoring service, and so on.

A number of algorithms for artifact detection and management are provided herein. The algorithms are based on a variety of different techniques. For example, in one multi-modal algorithm embodiment, the algorithm for artifact elimination uses a sensor such as an accelerometer to identify a heartbeat movement and the actual QRS. Then any signal that is found to be synchronous with the QRS can be used to help direct the search for the next QRS which may be embedded in signal noise, and those signals not synchronous with the QRS can be filtered.

The health parameter data collected from sensors on a patient can be communicated from a wearable monitor device to data collection and analysis systems in a variety of modes. In some implementations, the monitor device can wirelessly transmit data to a cellular telephone that is coupled via a short-range wireless link to a transceiver (e.g., Bluetooth, RF, infrared, etc.), and the transceiver can communicate over a network such as the internet to a remote monitoring server. In some implementations, a control module from the wearable monitor device can be decoupled from the monitor device and coupled to a base station, computing device, docking device coupled to a computing device, and the like. The health parameter data can then be downloaded from the control module to the base station, and the base station can communicate the data to a remote monitoring server over a network (including, for example, internet, Ethernet, telephone landline or cellular phone networks). In some embodiments, a combination of such techniques and other techniques well known in the art can be used to communicate the health parameter data collected by the monitor device to a remote location for data monitoring and analysis.

The sensors used in the monitoring devices provided herein can include a variety of types and configurations. Some sensors are non-invasive. That is, some sensors make contact with the skin surface of the patient. Other sensors penetrate the dermal layers of the patient. Such penetrating sensors may also be referred to herein as "microneedles" or "microsensors." Microneedles can advantageously eliminate signal interference from the patient's skin in some circumstances. Therefore, microneedles may provide enhanced signal reception for parameters including but not limited to electrocardiography (ECG), electroencephalography (EEG), electromyography (EMG), and others.

The monitoring devices provided herein may be used to collect other data types including but not limited to blood pressure, weight, hip waist ratio, oximetry, thoracic, bioimpedance, physical activity, temperature, drug levels, microfluidics (including serum and urine analytes and protein-based assays), respiration rate, heart sounds, voice recordings, heart rate (heart rate), posture, analyte values such as blood glucose, just to provide a few more examples. Movement or activity may be sensed with appropriate accelerometers or multi-axis gyroscopes, such as micro electromechanical system (MEMS) devices. Such collected data may in turn be synthesized using various algorithms to calculate other health status indicators such as QRS complex values, RR intervals, PVC values, arrhythmia, P wave, and others.

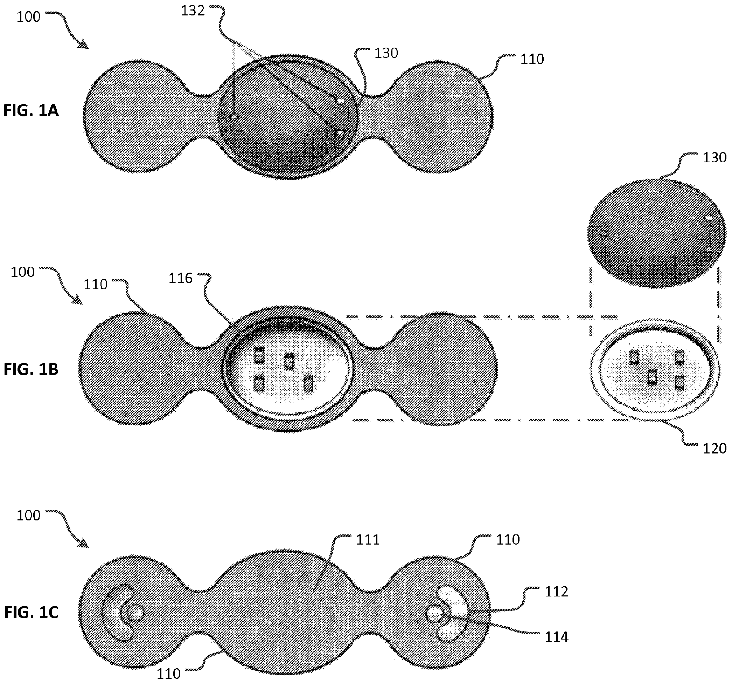

Figures 1A-1C provide an example wearable modular external monitoring device 100 shown in a top view (Figure 1A), an exploded top view (Figure 1B), and a bottom view (Figure 1C). The modular external monitoring device 100 depicted includes a sensor patch 110, control unit 120, and a snap-on monitor 130. Sensor patch 110 includes a central cradle 116 that is a receptacle for releasably receiving control unit 120. With control unit 120 installed in cradle 116, snap-on cap 130 can be installed onto sensor patch 110 over control unit 120 to detain control unit 120 in sensor patch 110 as shown in Figure 1A. Snap-on cap 130 can engage with complementary physical features on the sensor patch 110 so as to snap in place using a mechanical fit, for example. In some embodiments, snap-on cap 130 engages with sensor patch 110 to create a water-resistant seal therebetween.

When control unit 120 is installed in sensor patch 110, electrical connections are made such that control unit 120 is in electrical communication with the sensors that are visible on the bottom of sensor patch 110. Sensor patch 110 includes, in this example embodiment, an ECG electrode 112 and a bioimpedance sensor 114. However, a wide variety of types, configurations, and numbers of sensors can be included in sensor patch 110 as described further herein, and as known in the art.

In addition, in some embodiments a GPS system can be included in control unit 120. The inclusion of GPS in monitoring device 100 can be advantageous in multiple ways. First, as will be described further herein, the patient's geographical location and movements as determined by the GPS system can be used as a factor for establishing an expected baseline heart activity while the patient is at certain locations and/or undergoing certain movements. Further, GPS can be used to help locate the patient should the patient become indisposed for whatever reason, including a cardiac event or an unrelated event such as a motor vehicle accident. In addition, in combination with accelerometers, the GPS will help define the total distance moved physically by the patient (e.g., when walking, running, using stairs, etc.) as opposed to other means of moving, such as in a vehicle or elevator, for example. Still further, by using GPS to define certain patient movements as from a vehicle or elevator, for example, extraordinary monitoring signals can be potentially attributed to artifact from the interference caused by the vehicle or elevator-related patient movements.

Some portions of modular external monitoring device's 100 sensory and monitoring systems are located in sensor patch 110, and other portions are located in control unit 120 and snap-on cap 130. For example, in this embodiment sensor patch 110 includes the sensor devices, such as ECG electrode 112 and bioimpedance sensor 114. A power source such as a battery (not shown), and electrical contacts that mate with complementary contacts on control unit 120 can also be included in the sensor patch 110. Control unit 120 can include microelectronics including but not limited to a CPU, data storage memory, wireless transceiver, power management circuitry, sensor interface circuitry, alarm devices, and complementary contacts that mate with sensor patch 110 and snap-on cap 130. Snap-on cap 130, in addition to contacts that mate with control unit 120, can include user interface devices such as LEDs, a numeric display, a text display, an icon display, audio alarm devices, visual alarm devices, and a combination of such user interface devices.

Sensor patch 110 can be made from compliant polymeric materials and can have an adhesive on a bottom surface 111. In some embodiments, sensor patch 110 can comprise a material that is well-suited for the convenient placement on the patient's skin, consistent retention thereon, and non-irritating skin contact. For example, sensor patch 110 can comprise a soft elastomer such as a thermoplastic elastomer, silicone, or the like.

Snap-on cap 130 can include indicator LEDs 132 (or another type of user interface). LEDs 132 can signal to the patient various messages such as errors, the proper functioning of monitoring device 100, if the monitoring device 100 is transmitting data, and the like. Snap-on cap 130 can be a polymeric material. In some cases, snap-on cap 130 is a more rigid material than sensor patch 110. For example, snap-on cap 130 can be made from any suitable material including but not limited to polypropylene, polystyrene, acrylonitrile butadiene styrene (ABS), polycarbonate, PVC, silicone, or the like.

As best seen in FIG. 1B, control unit 120 that includes the memory, CPU, communications, etc. can be reversibly attached/detached to sensor patch 110. Because of this arrangement, a particular control unit 120 can be used with multiple properly configured sensor patches 110, and conversely, multiple properly configured control units 120 can be used with a particular sensor patch 110. In one example scenario of operating monitoring device 100, a patient can be given two control units 120 that are programmed and personalized identically. The control units 120 are rotated daily. That is, each day the patient removes a control unit 120 and installs the other control unit 120. The following day, the patient repeats the process-again swapping control units 120. In this manner, a particular control unit 120 gets used every other day. This usage of control units 120 can be independent of the patient's frequency of replacing sensor patches 110.

The control unit 120 that is removed from sensor patch 110 and is not in use on a particular day is installed into communication with a base station computer system. The base station can be located in the patient's home, at a treatment site, or a combination of such locations. The base station has network access (wired or wirelessly) and a standard AC power supply. In some cases, a cellular phone or other portable computing device can be used instead of the base station. The base station then downloads the health parameter data from control unit 120 and either stores the data to the data storage system of the base station or transmits the data to a monitoring service via the network. Further analysis of the data can be performed by the base station, monitoring service, and by health practitioners using the systems. Data can be presented graphically. Trends can be compiled and displayed for analysis. Various types of algorithms can be applied to provide artifact management, arrhythmia detection and other types of data analysis and diagnostic tools.

While control unit 120 typically downloads the health parameter data to a base station or equivalent device, in some cases control unit 120 while installed in the sensor patch 110 can send wireless transmissions to the base station or over cellular networks based on triggering events. Such triggering events can be determined for a particular patient and programmed into control units 120 for the patient. For example, a triggering event may be a particular variability in RR over a short time period, or an ECG QRS morphology, or the like.

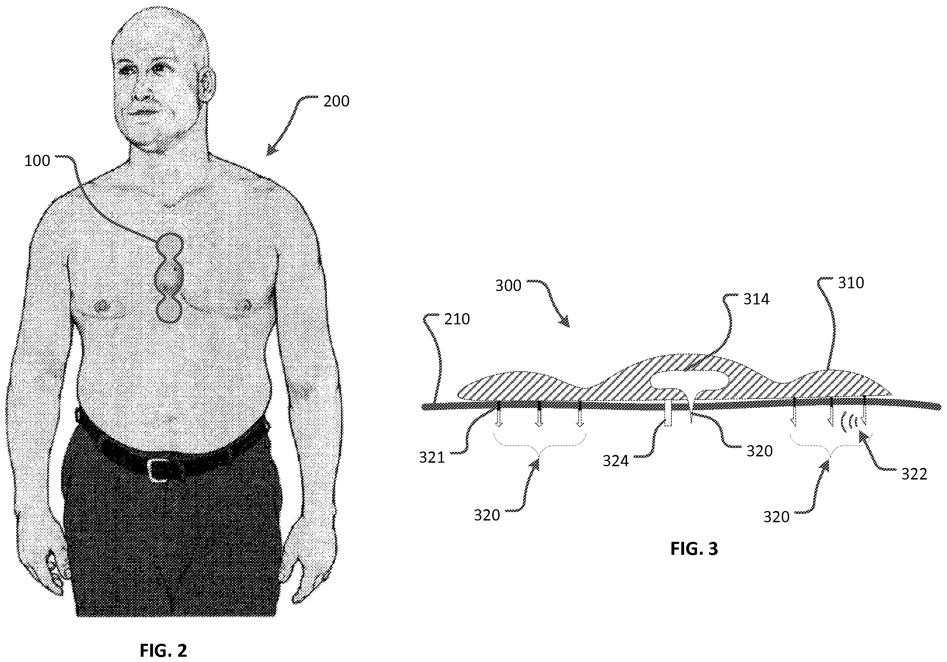

Referring to Figure 2, a patient 200 is illustrated wearing modular external monitoring device 100. Monitoring device 100 is adhered to the skin of patient 200. In this example, monitoring device 100 is on the chest of patient 200 in a position over the sternum to measure heart and respiratory health parameters. This position is less prone to motion artifact than some other locations, because the skeletal and muscle motion above the sternum is generally minimal. Still, in other implementations monitoring device 100 is worn on other areas of patient 200. For example, monitoring device 100 may be worn on the head, abdomen, back, side, extremities, and other suitable locations on patient 200. The location of monitoring device 100 on patient 200 will depend on the type of health parameter data to be collected.

Referring to Figure 3, a cross-sectional side view of another example modular external monitoring device 300 is depicted on the skin 210 of a patient. Monitoring device 300 includes microneedles 320 that can be employed as sensors, injection devices, sampling devices, and for other like purposes. Microneedles 320 can be barbed or otherwise include structures which facilitate adherence to skin 210.

Microneedles 320 penetrate the skin 210 and the distal tips of the mocroneedles 320 reside subdermally. Therefore, microneedles 320, when used as sensors, have enhanced signal reception (e.g., for ECG, EEG, EMG, etc.). The enhanced reception can be due to the elimination of "shielding" by dermal layers to outside interference as well as because of closer proximity to organ to be monitored. In another implementation, microneedles 320 have access to interstitial fluid for sensing electrolytes, glucose, oxygen, pH, temperature, and so on. The portions of microneedles 320 near sensor patch 310 can be insulated portions 321 such that the only electrical recording would come from the exposed electrodes at the distal end of microneedles 320 that are positioned deeper into the tissue. In some cases, this arrangement can reduce signal artifact caused by patient motion or from intermittent contact between skin 210 and a surface electrode (e.g., electrodes 112 and 114 of Figure 1C). Further, there are known electrical potentials that arise from the surface of skin 210 which can be a source of electrical noise. The avoidance of recording from the surface of skin 210 can decrease or eliminate this source of electrical noise.

In some examples, microneedles 320 can alternatively be used for drug delivery by injecting medication from a reservoir 314 located within or coupled to sensor patch 310. For example, a drug such as a steroid, lidocain, and others can be beneficially administered to the patient to prevent discomfort and inflammation which could otherwise result from the chronic use of sensor patch 310 and microneedles 320. In another example, an agent can be delivered from reservoir 314 through microneedles 320 to treat a patient's particular detected disorder. Drugs such as quinidine, beta-blocker, amiodarone, insulin, and so on can be used in such applications.

Microneedles 320 include accelerometers at distal tips to help with the control of signal noise from the sensors. For example, movement sensed at microneedle 320 tip by an accelerometer can indicate motion and typical signal noise associated with such motion can be anticipated and managed. In some cases,
electrical circuitry or software can be used for cancelation, correction, and filtering of the resulting signal to thereby reduce motion artifact. Previous attempts to record signal noise using accelerometers at locations removed from the recording electrode - even by a small amount - have been ineffective due to the lack of correlation between the forces at the accelerometer and at the electrode. An accelerometer in microneedle 320, or at the base of the microneedle 320, can resolve that problem.

In some examples, monitor device 300 can also include one or more piezoelectric sensors 324. Piezoelectric sensors 324 can be used to measure bioimpedance which can in turn provide a useful signal for artifact elimination, arrhythmia detection, determination of respiration rate, and other purposes.

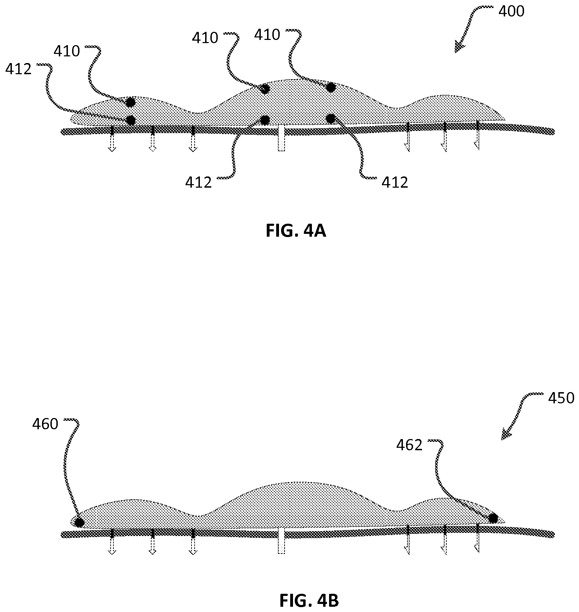

In reference to Figure 4A, a modular external monitoring device 400 is illustrated including one or more upper accelerometers 410 and one or more lower accelerometers 412. In some embodiments, one or more multi-axis gyroscopes can be used in addition to or as a substitute for accelerometers 410 and 412.

Including accelerometers 410 and 412 in monitoring device 400 can provide many advantages. In some embodiments, monitoring device 400 only captures or analyzes data when motion levels as determined by accelerometers 410 and 412 are below certain thresholds levels (so as to avoid motion induced artifact). Accelerometers 410 and 412 can be oriented in multiple arrangements to facilitate several functions (e.g. physiologic monitoring and device context determination). For example accelerometers 410 and 412 can be incorporated into the monitoring device 400 platform as independent sensors, or into the electrodes themselves (as described herein). Integration of motion data at the electrode interface may be beneficial when correlated with motion at a distance - away from the electrodes -this would allow for noise subtraction due to motion of monitoring device 400.

Further uses for accelerometers 410 and 412 can include physiologic monitoring. For example, physical activity or inactivity - including "learned" activities, can be measured, and correlations of these learned activities with expected changes in other monitored/sensed data inputs can be used to enhance the value of the data collected by monitoring device 400. Signals from accelerometers 410 and 412 can be used to indicate patient falls, long-term inactivity, and levels of activity. Heart sounds and motion permitting event timing and ECG can be detected by accelerometers 410 and 412 in some embodiments. Respiration can be determined based on motion of monitor device 400, bioimpedance changes, or both. Accelerometers 410 and 412 can also be used to determine erect or supine posture. All of these measurements can be combined and cross-checked to determine the presence of artifact and/or increase the sensitivity and specificity of event recording.

Signal noise (artifact) can be very difficult to distinguish from potentially dangerous and rapid heart rhythms. Artifact may be caused by a number of conditions, with the two primary ones being (1) mechanical motion with subsequent myopotentials and (2) poor electrode contact. With poor electrode contact, bioimpedance data may be useful particularly when supplemented with data from accelerometers 410 and 412. With regard to physical motion, accelerometers 410 and 412 can be useful for determining that artifact is present secondary to motion data from accelerometers 410 and 412. The physical motion that results in ECG artifact can have a characteristic "signature" unique to a specific activity such as walking and other routine activities, such as tremor, or local skeletal muscle contraction. Thus, rather than performing artifact rejection, the monitoring device 400 can be programmed to detect artifact and classify it as such using the "noise signature" that results from characteristic ECG signals. These unique activity signatures can be taught to the control unit during registration and thereby enhance signal quality and event detection, by artifact detection and rejection.

The location of the accelerometers 410 and 412 can be advantageously selected to enable artifact signal noise detection. Integration of accelerometers onto electrodes as described herein provides one beneficial location. For example, in some embodiments a first accelerometer is embedded in a microneedle or other type of sensor, and a second accelerometer is located in the sensor patch (such as on a circuit board of the control unit). Analysis of the relative motion of these two accelerometers would be useful in developing these characteristic "signatures" of particular types of motion. For example, an individual's stride during walking would likely result in similar motion detected by accelerometers in contact with the skin and by accelerometers on the circuit board, whereas as the type of motion associated with myopotentials (e.g., pectoralis motion) or vibratory motion from riding in a car on a bumpy road may result in different signals from the two accelerometers. If these "noise" motion signals occur in the setting of "high heart rates" it can indicate that artifact is likely present. For example, radial motion such as may be expected with a swinging of the arms or movement of the pectoralis would result in a greater translational motion of the upper accelerometer 410 than a lower accelerometer 412. This difference can be taken advantage of to define a characteristic signature to each type of motion and thus identify specific activities. For example, if a person is traveling in a car and not otherwise moving, then accelerometer 410 would equal accelerometer 412 as both accelerometers 410 and 412 are being equally translated by the car's activity. In contrast, if a person is actively rowing a boat, then accelerometer 410 would be greater than accelerometer 412, thus providing for the detection of this type of activity.

Accelerometer 410 and 412 data in combination with other data such as ECG and impedance data can also be used to indicate poor contact of monitoring device 400 with the patient. In turn, monitoring device 400 can alert the patient or other personnel, reject certain data signals, and so on. The user of multiple accelerometers 410 and 412 can be used to enhance this function. For example, if signals from accelerometers 410 shows motion analogous to accelerometers 412 then skin contact may be lost. Accelerometer 410 motion should be less than that of accelerometer 412 when monitor device 400 is correctly placed and in correct skin contact.

In reference to Figure 4B, a modular external monitoring device 450 is illustrated including one or more first end accelerometers 460 and one or more second end accelerometers 462. In some embodiments, one or more multi-axis gyroscopes can be used in addition to or as a substitute for accelerometers 460 and 462.

Similar to the functionality of accelerometers 410 and 412 as described herein, the relative motion between accelerometers 460 and 462 can indicate monitor device 450 that is being twisted or otherwise configured. Such a motion could indicate a poor contact with the patient's skin such as a detachment of a "wing" or end portion of monitoring device 450.

In some examples, a combination of vertically differentiated accelerometers (410 and 412) and horizontally differentiated accelerometers (460 and 462) can be used on a single monitoring device. In addition, other sensors such as a temperature sensor embedded near an electrode can be included to enhance detection of improper placement or detachment of monitoring device 450 from the patient's skin. For example, a temperature sensor may indicate a sudden change in temperature or sudden drop in temperature that indicates poor electrode contact with the patient's skin.

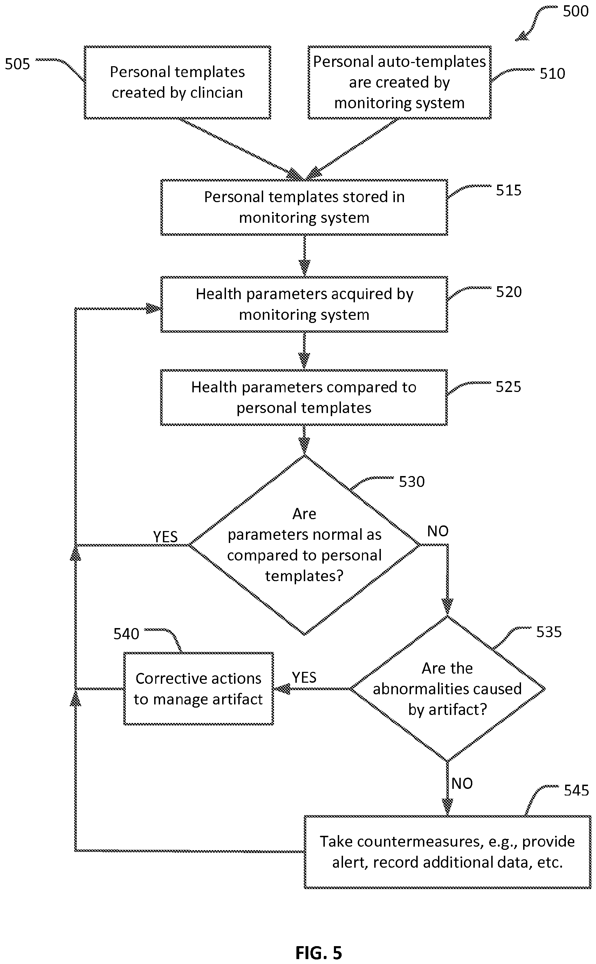

Figure 5 relates to an exemplary method of operating the health parameter monitoring system. Referring to Figure 5, personal templates can be created and used to identify health monitoring events and artifact in accordance with an example personal template algorithm 500 as shown. These templates can be used to "personalize" the device to the user-patient. Specific signal patterns associated with the patient and the patient's interactions with the monitoring device can be input into templates that are stored in the monitoring system's memory. For example, personal characteristics such as the patient's anatomy (e.g., fat, tissue motion, muscle interference, gait, etc.), the patient's environment, and the patient's common day-to-day activities can be incorporated into the personal templates.

In general, this personal template algorithm 500 is used to establish a change from normal for a particular patient. Algorithm 500 can be performed by a health parameter monitoring system that is programmed with the logic described herein. The algorithm may be run on the CPU of the wearable portion of the monitoring system, or on a portable computing device (e.g., a cell phone, tablet computer, PDA, etc.), or on a base station, or on a computing device at a remote monitoring service, and the like, or using a combination of such computing devices.

The personal template algorithm 500 operates in general as follows. Health parameter data or combinations of health parameter data can be compiled for a patient using a remote health parameter monitoring system. Various types of physiologic biometric data such as ECG, QRS morphology, QRS amplitude, QRS duration, PR interval, impedance, and the like can be used. Extra-physiologic inputs (e.g., pollen, temperature, smog, geographic location, season, humidity) can also be combined with the health parameter data to add context. Personal templates can be compiled from the data. The personal templates can be created by a clinician or by the monitoring system automatically. The personal templates can be stored in the memory of the monitoring system. Real-time-measured health parameters can be compared with the personal templates to determine if differences between the real-time parameters and the personal templates exist. If noteworthy differences exist, the real-time parameter data can be analyzed to determine if the differences are attributable to artifact signal noise. If artifact is not determined to be the cause the differences, the monitoring system can classify the situation as a health event.

At operation 505, personal templates of health parameter data for the patient are created by a clinician. In some cases, the data for the personal template can be acquired from the patient's monitoring system, but in some cases other devices are used for collecting the data for the personal template. For example, in some instances a standard in-office 12-lead ECG system, and other such clinical devices, can be used to collect the data to create the personal template. The templates can include parameters and combinations of parameters including but not limited to QRS, heart rate, heart sounds, pulmonary data such as impedance, bold oxygenation, activity, respiration frequency, blood pressure, analyte values (e.g., blood glucose), weight, and so on. The clinician can input the template data into the monitoring system for storage and comparison purposes (as will be explained further herein). These data could be optionally sub-stratified based on time of day, activity level, and other factors that add context to the templates.

At operation 510, personal auto-templates of health parameter data for the patient are automatically created by the monitoring system. Any of a variety of events may trigger the monitoring system to create the personal auto-templates. For example, when a new sensor patch is installed the monitoring system may create a personal auto-template. Or, when a sensor patch is repositioned the monitoring system may create a personal auto-template. The creation of the auto-templates can also take place on a periodic time basis, such as every 12 hours, daily, every other day, and so on. In another example, an auto-template can be created whenever the monitoring system is powered on, e.g., during system start-up. In some cases, a remote monitoring service can initiate the creation of an auto-template using communications via a network from the service to the patient's monitor.

In some examples, various personal templates can be combined and correlated to each other. For example, personal auto-templates can be combined with personal templates created by a clinician, or a clinician can modify an auto-template. In addition, the dynamic interaction between two or more templates can provide additional information for analysis of disease. For example, heart rate is related to respiratory rate and activity. Therefore, those three templates can be related together and cross-referenced. For example, it is expected that the respiratory rate is high when the heart rate and/or activity level is high, but if the respiratory rate and/or heart rate is high when the activity level is low, a possible disease condition may exist (e.g., asthma, etc.). Higher order integrated templates of multiple physiologic parameters can be created through this technique to detect alterations in homeostasis for a given patient, and for that patient over time (during disease development, etc.).

The process of compiling the personal auto-templates by the monitor can include signal averaging. In one example, the monitor's control unit can verify no motion is occurring (e.g., a supine position indication from an accelerometer or minimal motion indication from the accelerometer). The monitor can then capture multiple signals (e.g., 100 QRS complexes). The signals can be averaged and stored. This process can be used to establish a personal template with reduced noise and enhanced accuracy.

The process of compiling the personal auto-templates by the monitor can also include a context of the patient. Therefore, a particular health parameter can have multiple templates that are distinguish from each other based on the context of the patient. For example, contexts can include walking, running, movement causing sensor patch flexure, sensor detachment, external auditory noise, car travel, signal strength decreases, and the like.

At operation, 515 the personal templates are stored in the monitoring system. The storage location can be, for example, in the memory located in the wearable component, on a computing system at the patient's home (such as a base station), a portable computing device of the patient (a cell phone, laptop computer, tablet computer, PDA, etc.), or at a remote computing system (e.g., at a monitoring service, cloud-based servers, hospital system, and so on).

At operation 520, the monitoring system operates to acquire the patient's health parameters as described herein. The monitor, in addition to capturing health parameters, can use sensory data to determine a context of the patient. For example, the monitor can determine if the patient has a high activity level (e.g., running) based on heart rate data or respiration rate data. In some embodiments, the determined context can be used as a factor for determining what personal template to compare the real-time acquired health parameter data to.

At operation 525, the real-time acquired health parameter data is compared to the personal templates. In some cases, the data is combined and cross-referenced as a part of the comparison process. That is, multiple sensed data can be correlated and multidimensional plots can be created of these parameters over time.

At operation 530, the monitoring system determines whether the real-time acquired health parameter data is normal, or within an expected range, as compared to the personal templates. In some examples, a range of values around the templates can be established as an expected range. For example, if the patient's average heart rate is 80 beats per minute, an expected range of about 60 to 100 beats per minute can be established. In other examples, factor of about +/- 10%, 15%, 20%, 25%, and so on can be used in relation to the baseline template values to establish an expected range.

If the monitoring system determines the real-time acquired health parameter data is normal as compared to the personal templates, the personal template algorithm 500 proceeds to monitoring health parameters in operation 520. However, if the monitoring system determines the real-time acquired health parameter data is not normal as compared to the personal templates, the personal template algorithm 500 proceeds to operation 535.

At operation 535, the algorithm determines whether the abnormalities of the real-time acquired health parameter data as compared to the personal templates is a result of artifact signal noise. Examples of algorithms to identify the presence of artifact are provided elsewhere in this document. If operation 535 determines that the abnormalities of the real-time acquired health parameter data as compared to the personal templates is a result of artifact signal noise, the personal template algorithm 500 proceeds to operation 540. At operation 540, the algorithm takes corrective actions to eliminate, reduce, or otherwise manage the artifact. Example techniques to eliminate, reduce, or otherwise manage artifact are provided elsewhere in this document. However, if operation 535 determines that the abnormalities of the real-time acquired health parameter data as compared to the personal templates are not a result of artifact signal noise, the personal template algorithm 500 proceeds to operation 545.

At operation 545, in response to finding that the abnormalities of the real-time acquired health parameter data as compared to the personal templates are not a result of artifact signal noise, the algorithm 500 can trigger the monitoring system to take countermeasures related to a determination by the algorithm that the patient is experiencing a health event. The countermeasures can be a variety of actions, e.g., providing an alarm message to the user, providing an alarm message to a remote monitoring system, providing a message to the user to confirm proper functioning of the wearable monitoring component, recordation of additional types of data, recordation of data more frequently, additional data analysis, and so on. Acute or chronic changes to the personal templates may be used to prompt patient contact and inquiries from the monitoring service or physician.

In regard to multidimensional plots of parameters, if over a period of time the configuration the multidimensional plot changes, an alert can be generated -including identifying a specific perturbation such as higher blood pressure, or lower heart rates, or higher weights versus a non-specific more subtle perturbation only apparent after cross correlation. This technique can be predictive of a more specific perturbation before it becomes overtly manifest, such as subtle changes of heart rate and weight (not immediately outside predefined parameters) but when taken together suggesting early heart failure decompensation for example.

Another example technique for personalization of monitoring systems includes a biometric identifier algorithm. This biometric identifier algorithm can be initiated and activated at various times including during set-up, start-up, or restarting of the monitoring system. In one example, the device acquires and stores certain biometric data of the patient to whom the monitoring system is prescribed. Such biometric data can include but is not limited to ECG QRS morphology, QRS amplitude, QRS duration, PR interval, impedance, finger prints, skin markings such as freckles (e.g., using photo recognition), retinal scan, facial recognition, voice recognition, and so on. These parameters provide unique patterns to the individual patient. These biometrics patterns can be used for the purposes of routinely querying the user and verifying that the device is attached to the correct individual and to ensure personalization.

In another example algorithm, a multi-modal algorithm for artifact detection and management is provided. This algorithm utilizes multiple types of physiologic inputs (e.g., heart rate, ECG, respiration, oximetry, activity, posture, movement, etc.) from multiple sensors (accelerometers, gyroscopes, electrodes, bioimpedance sensors, microneedles, temperature sensors, audio sensors, etc.) to verify and eliminate noise, corroborate signals, reject artifacts, detect events, trigger recordings, and so on. The multi-modal approach can be used to increase the rate of artifact detection (and elimination) thereby improving the overall performance of the monitor system.

Using the multi-modal algorithm, the CPU in the wearable monitor (or another computer system in the overall monitoring system) would be able to select between sensors and algorithms to determine which signal is providing the best data stream. Alternatively, the control unit can use multiple sensors and algorithms to verify or corroborate signals to ensure fidelity and prevent false positives or false negatives in event detection. This approach would allow for cross-checking for artifact noise and signal quality. For example, if the ECG has noise in the heart rate signal, that could be compared with the impedance generated heart rate signal in an attempt to create a true signal for analysis (or rejection). The comparison may also result in confirming that the suspected artifact noise is actually occurring throughout the system and that it is not artifact. In that situation, the signals would be recorded for review by the monitoring service or physician.

This multi-modal algorithm approach can also be used to capture relevant data from one sensor when another is suffering from excess noise. For example, ECG sensors could be used to gather respiration data if accelerometers have high noise levels in their detection of respiration.

In some examples, various physiological signals can be integrated with each other using factors to weight these signals based on the quality of the acquisition. For example, signals such as QRS and ECG, bio-impedance, accelerometer, and sound can be combined, while weighting the highest quality signals the most, to define an accurate representation of the heartbeat. The eventual decision as to what and where the heart beat occurs, and hence definition of heart rate, will therefore be based on multiple inputs, with the greatest credibility given to the clearest (or least disrupted) signal. For each sensor input, a signal quality determination can be made and those sensor inputs with a high quality signal can be sent to the control unit for processing (e.g., entered into an arrhythmia detection algorithm), whereas those with a poor quality signal can be labeled as "noise" and sent to the control unit for elimination or other types of signal management (e.g., entered into a personal template algorithm or artifact algorithm).

In some examples of the multi-modal algorithm, the variety of sensor inputs (e.g., ECG, impedance, accelerometer, oximeter, etc.) can be weighted in terms of most valuable depending on the physiologic parameter to be measured (e.g., ECG is high for heart rate and rhythm, whereas impedance for heart rate and rhythm would be weighted lower).

The scope of the invention is defined by appended claims 1-6.

## Claims

1. A health parameter monitoring system comprising:
a sensor patch (110) adapted to be in contact with a human skin (210) surface, the sensor patch (110) comprising a plurality of sensors for measuring physiological or pathological parameters of a user (200), wherein the plurality of sensors are implemented as microneedles (320) comprising accelerometers present at distal tips, for filtering signal noise present in captured data;
a control unit (120), wherein the control unit is releasably receivable in a cradle (116) of the sensor patch (110), and wherein the control unit (120) is in electrical communication with the plurality of sensors when the control unit (120) is in the cradle (116);
a cap (130), wherein the cap (130) is configured to releasably couple with the sensor patch (110) to detain the control unit (120) in the cradle (116), and wherein the cap (130) includes a user interface that is configured to provide indications related to the functioning of the health parameter monitoring system; and
a biometric interface, wherein the biometric interface is configured to detect a biometric characteristic of the user (200), of the health parameter monitoring system.

2. The system of claim 1, wherein, in response to detecting a particular biometric characteristic of the user (200) the health parameter monitoring system will operate, and in response to not detecting the particular biometric characteristic of a user (200) the health parameter monitoring system will not operate.

3. The system of claim 2, wherein the particular biometric characteristic is a fingerprint.

4. The system of claim 2, wherein the particular biometric characteristic is a QRS morphology.

5. The system of claim 2, wherein the particular biometric characteristic is a bioimpedance.

6. A method of operating a health parameter monitoring system, the method comprising:
providing the health parameter monitoring system, the system comprising:
a sensor patch (110) adapted to be in contact with a human skin (210) surface, the sensor patch (110) comprising a plurality of sensors for measuring physiological or pathological parameters of a user (200), wherein the plurality of sensors are implemented as microneedles (320) comprising accelerometers present at distal tips, for filtering signal noise present in captured data;
a control unit (120), wherein the control unit (120) is releasably receivable in a cradle (116) of the sensor patch (110), and wherein the control unit (120) is in electrical communication with the plurality of sensors when the control unit (120) is in the cradle (116);
a cap (130), wherein the cap (130) is configured to releasably couple with the sensor patch (110) to detain the control unit (120) in the cradle (116), and wherein the cap (130) includes a user interface that is configured to provide indications related to the functioning of the health parameter monitoring system; and
a biometric interface, wherein the biometric interface is configured to detect a biometric characteristic of the user (200) of the health parameter monitoring system;
detecting a particular biometric characteristic of the user (200);
determining whether the detected particular biometric characteristic is sufficiently similar to a template of the particular biometric characteristic; and
allowing the health parameter monitoring system to operate in response to determining that the detected particular biometric characteristic is sufficiently similar to the template of the particular biometric characteristic, or not allowing the health parameter monitoring system to operate in response to determining that the detected particular biometric characteristic is not sufficiently similar to the template of the particular biometric characteristic.

## Patentansprüche

1. System zur Überwachung eines Gesundheitsparameters, umfassend:
ein Sensorpflaster (110), das dazu geeignet ist in Kontakt mit einer menschlichen Haut- (210) Oberfläche zu sein, wobei das Sensorpflaster (110) eine Vielzahl an Sensoren zum Messen physiologischer oder pathologischer Parameter eines Benutzers (200) umfasst, wobei die Vielzahl an Sensoren als Mikronadeln (320) ausgebildet ist, umfassend Beschleunigungsmesser, die an distalen Spitzen vorliegen, für ein Filtern von Signalrauschen, das in erfassten Daten vorhanden ist;
eine Steuer-/Regeleinheit (120), wobei die Steuer-/Regeleinheit lösbar in einer Halterung (116) des Sensorpflasters (110) aufnehmbar ist und wobei die Steuer-/Regeleinheit (120) mit der Vielzahl an Sensoren in elektrischer Verbindung steht, wenn sich die Steuer-/Regeleinheit (120) in der Halterung (116) befindet;
eine Kappe (130), wobei die Kappe (130) dazu eingerichtet ist, mit dem Sensorpflaster (110) lösbar gekoppelt zu sein, um die Steuer-/Regeleinheit (120) in der Halterung (116) zu halten, und wobei die Kappe (130) eine Benutzerschnittstelle umfasst, die dazu eingerichtet ist, Angaben in Bezug auf die Funktion des Systems zur Überwachung eines Gesundheitsparameters bereitzustellen; und
eine biometrische Schnittstelle, wobei die biometrische Schnittstelle dazu eingerichtet ist, eine biometrische Eigenschaft des Benutzers (200) des Systems zur Überwachung eines Gesundheitsparameters zu erfassen.

2. System nach Anspruch 1, wobei als Reaktion auf ein Erfassen einer bestimmten biometrischen Eigenschaft des Benutzers (200) das System zur Überwachung eines Gesundheitsparameters arbeiten wird und als Reaktion auf ein Nichterfassen der bestimmten biometrischen Eigenschaft eines Benutzers (200) das System zur Überwachung eines Gesundheitsparameters nicht arbeiten wird.

3. System nach Anspruch 2, wobei die bestimmte biometrische Eigenschaft ein Fingerabdruck ist.

4. System nach Anspruch 2, wobei die bestimmte biometrische Eigenschaft eine QRS-Morphologie ist.

5. System nach Anspruch 2, wobei die bestimmte biometrische Eigenschaft eine Bioimpedanz ist.

6. Verfahren zum Betreiben eines Systems zur Überwachung eines Gesundheitsparameters, wobei das Verfahren umfasst:
Bereitstellen des Systems zur Überwachung eines Gesundheitsparameters, wobei das System umfasst:
ein Sensorpflaster (110), das dazu eingerichtet ist in Kontakt mit einer menschlichen Haut- (210) Oberfläche zu sein, wobei das Sensorpflaster (110) eine Vielzahl an Sensoren zum Messen physiologischer oder pathologischer Parameter eines Benutzers (200) umfasst, wobei die Vielzahl an Sensoren als Mikronadeln (320) ausgebildet ist, umfassend Beschleunigungsmesser, die an distalen Spitzen vorliegen, für ein Filtern von Signalrauschen, das in erfassten Daten vorhanden ist;
eine Steuer-/Regeleinheit (120), wobei die Steuer-/Regeleinheit (120) lösbar in einer Halterung (116) des Sensorpflasters (110) aufnehmbar ist und wobei die Steuer-/Regeleinheit (120) mit der Vielzahl an Sensoren in elektrischer Verbindung steht, wenn sich die Steuer-/Regeleinheit (120) in der Halterung (116) befindet;
eine Kappe (130), wobei die Kappe (130) dazu eingerichtet ist, mit dem Sensorpflaster (110) lösbar gekoppelt zu sein, um die Steuer-/Regeleinheit (120) in der Halterung (116) zu halten, und wobei die Kappe (130) eine Benutzerschnittstelle umfasst, die dazu eingerichtet ist, Angaben in Bezug auf die Funktion des Systems zur Überwachung eines Gesundheitsparameters bereitzustellen; und
eine biometrische Schnittstelle, wobei die biometrische Schnittstelle dazu eingerichtet ist, eine biometrische Eigenschaft des Benutzers (200) des Systems zur Überwachung eines Gesundheitsparameters zu erfassen;
Erfassen einer bestimmten biometrischen Eigenschaft des Benutzers (200);
Bestimmen, ob die erfasste bestimmte biometrische Eigenschaft ausreichend ähnlich zu einer Vorlage der bestimmten biometrischen Eigenschaft ist; und
Zulassen, dass das System zur Überwachung eines Gesundheitsparameters als Reaktion auf ein Bestimmen, dass die erfasste bestimmte biometrische Eigenschaft ausreichend ähnlich zu der Vorlage der bestimmten biometrischen Eigenschaft ist, arbeitet, oder Nichtzulassen, dass das System zur Überwachung eines Gesundheitsparameters als Reaktion auf ein Bestimmen, dass die erfasste bestimmte biometrische Eigenschaft nicht ausreichend ähnlich zu der Vorlage der bestimmten biometrischen Eigenschaft ist, arbeitet.

## Revendications

1. Un système de surveillance de paramètres de santé comprenant:
un patch capteur (110) adapté pour être en contact avec une surface de peau humaine (210), le patch capteur (110) comprenant une pluralité de capteurs pour mesurer des paramètres physiologiques ou pathologiques d'un utilisateur (200), dans lequel la pluralité de capteurs sont mis en oeuvre sous forme de microaiguilles (320) comprenant des accéléromètres présents aux extrémités distales, pour filtrer le bruit du signal présent dans les données capturées;
une unité de commande (120), dans lequel l'unité de commande est recevable de manière amovible dans un berceau (116) du patch capteur (110), et dans lequel l'unité de commande (120) est en communication électrique avec la pluralité de capteurs lorsque l'unité de commande (120) est dans le berceau (116);
un capuchon (130), dans lequel le capuchon (130) est configuré pour se coupler de manière amovible avec le patch capteur (110) pour retenir l'unité de commande (120) dans le berceau (116), et dans lequel le capuchon (130) inclut une interface utilisateur qui est configurée pour fournir des indications relatives au fonctionnement du système de surveillance de paramètres de santé; et
une interface biométrique, dans lequel l'interface biométrique est configurée pour détecter une caractéristique biométrique de l'utilisateur (200) du système de surveillance de paramètres de santé.

2. Système selon la revendication 1, dans lequel, en réponse à la détection d'une caractéristique biométrique particulière de l'utilisateur (200), le système de surveillance de paramètres de santé fonctionnera, et en réponse à la non détection de la caractéristique biométrique particulière d'un utilisateur (200), le système de surveillance de paramètres de santé ne fonctionnera pas.

3. Système selon la revendication 2, dans lequel la caractéristique biométrique particulière est une empreinte digitale.

4. Système selon la revendication 2, dans lequel la caractéristique biométrique particulière est une morphologie QRS.

5. Système selon la revendication 2, dans lequel la caractéristique biométrique particulière est une bioimpédance.

6. Procédé de fonctionnement d'un système de surveillance de paramètres de santé, le procédé comprenant:
fournir le système de surveillance de paramètres de santé, le système comprenant:
un patch capteur (110) adapté pour être en contact avec une surface de peau humaine (210), le patch capteur (110) comprenant une pluralité de capteurs pour mesurer des paramètres physiologiques ou pathologiques d'un utilisateur (200), dans lequel la pluralité de capteurs sont mis en œuvre sous forme de microaiguilles (320) comprenant des accéléromètres présents aux extrémités distales, pour filtrer le bruit du signal présent dans les données capturées;
une unité de commande (120), dans lequel l'unité de commande (120) est recevable de manière amovible dans un berceau (116) du patch capteur (110), et dans lequel l'unité de commande (120) est en communication électrique avec la pluralité de capteurs lorsque l'unité de commande (120) est dans le berceau (116);
un capuchon (130), dans lequel le capuchon (130) est configuré pour se coupler de manière amovible avec le patch capteur (110) pour retenir l'unité de commande (120) dans le berceau (116), et dans lequel le capuchon (130) inclut une interface utilisateur qui est configurée pour fournir des indications relatives au fonctionnement du système de surveillance de paramètres de santé; et
une interface biométrique, dans lequel l'interface biométrique est configurée pour détecter une caractéristique biométrique de l'utilisateur (200) du système de surveillance de paramètres de santé;
détecter une caractéristique biométrique particulière de l'utilisateur (200);
déterminer si la caractéristique biométrique particulière détectée est suffisamment similaire à un modèle de la caractéristique biométrique particulière; et
permettre au système de surveillance de paramètres de santé de fonctionner en réponse à la détermination que la caractéristique biométrique particulière détectée est suffisamment similaire au modèle de la caractéristique biométrique particulière, ou de ne pas permettre au système de surveillance de paramètres de santé de fonctionner en réponse à la détermination que la caractéristique biométrique particulière détectée n'est pas suffisamment similaire au modèle de la caractéristique biométrique particulière.
